# EUROPEAN PATENT APPLICATION

(11) **EP 3 988 574 A1**
(43) Date of publication of application: **27.04.2022**
(21) Application number: 20748159.9
(22) Date of filing: 03.02.2020
(51) Int. Cl.: C07K 16/46, C07K 16/32, C07K 14/82, C12N 15/12

(54) **ANTI-HER2 BISPECIFIC ANTIBODY AND APPLICATION THEREOF**

(30) Priority: 03.02.2019 CN 201910109008
(71) Applicant: Shanghai Bao Pharmaceuticals Co., Ltd., Shanghai 215123 (CN)
(72) Inventor: WANG, Zheng, Suzhou, Jiangsu 215123 (CN); XU, Yunxia, Suzhou, Jiangsu 215123 (CN)
(74) Representative: Zwicker, Jörk
(86) International application number: PCT/CN2020/074171
(87) International publication number: WO 2020/156555

(57) **Abstract**

Provided is an anti-HER2 bispecific antibody, comprising a first protein functional region and a second protein functional region for respectively recognizing extracellular domain II and extracellular domain IV of HER2. The first protein functional region comprises a first heavy chain and a first light chain. The second protein functional region comprises a second heavy chain and a second light chain. Also provided are pharmaceutical compositions comprising the bispecific antibody and an application thereof. The bispecific antibody and the pharmaceutical compositions containing the same can be used for cancer treatment.

## Description

The present application claims the priority of Chinese Patent Application No. 2019101090084 filed on February 3, 2019. The aforementioned Chinese patent application is incorporated in the present application by reference in its entirety.

### Technical Field

The present invention relates to the field of biomedicine, in particular to an anti-HER2 bispecific antibody and an application thereof.

### Background Art

HER2 is a 185 kDa member of the epidermal growth factor receptor (EGFR) family of cell surface receptor tyrosine kinases, and the EGFR family comprises four different receptors: EGFR/ErbB-1, HER2/ErbB-2, HER3/ErbB-3 and HER4/ErbB-4. The four members of the EGFR family form homodimers and heterodimers, and HER2 is the preferred one and the strongest dimerization partner of other ErbB receptors (Graus-Porta et al., Embo J 1997; 16: 1647-1655; and Tao et al., J Cell Sci 2008; 121: 3207-3217). HER2 may be activated by overexpression or by heterodimerization with other ErbBs that may be activated through ligand binding (Riese and Stern, Bioessays 1998; 20: 41-48). For HER2, no ligand has been identified yet. HER2 activation leads to receptor phosphorylation, which triggers downstream cascade signal amplification through multiple signaling pathways, such as MAPK, phosphoinositide 3-kinase/AKT, JAK/STAT and PKC, which ultimately results in the regulation of a variety of cellular functions, such as growth, survival and differentiation (Huang et al., Expert Opin Biol THER 2009; 9: 97-110).

Concerns about HER2 in tumors have focused on its role in breast cancer, where HER2 overexpression has been reported in approximately 20% of cases, and it has been associated with poor prognosis (Reese et al., Stem Cells 1997; 15: 1-8; Andrechek et al., Proc Natl Acad Sci USA 2000; 97: 3444-3449; and Slamon et al., Science 1987; 235: 177-182). In addition to breast cancer, HER2 expression has been also associated with other types of human cancer, including prostate cancer, non-small cell lung cancer, bladder cancer, ovarian cancer, gastric cancer, colon cancer, esophageal cancer and squamous cell carcinoma of the head and neck (Garcia de Palazzo et al., Int J Biol Markers 1993; 8: 233-239; Ross et al., Oncologist 2003; 8: 307-325; Osman et al., J Urol 2005; 174: 2174-2177; Kapitanovic et al., Gastroenterology 1997; 112: 1103-1113; Turken et al., Neoplasma 2003; 50: 257-261; and Oshima et al., Int J Biol Markers 2001; 16: 250-254). The table below shows the expression of HER2 in tumor cells from various sources.

**Table 1: Tumor cells and HER2 expression therein.**

| **No.** | **Cell name** | **Cell type** | **HER2 expression** |
|---|---|---|---|
| 1 | BT474 | Breast duct carcinoma | 3+ |
| 2 | NCI-N87 | Gastric cancer cells | 3+ |
| 3 | JIMT-1 | Trastuzumab-resistant breast cancer cells | 2+ |
| 4 | MDA-MB-175 | Breast duct carcinoma cells | 1+ |
| 5 | SK-BR-3 | Breast cancer cells | 3+ |
| 6 | Calu-3 | Lung cancer cells | 3+ |

Trastuzumab is a recombinant, humanized monoclonal antibody that targets domain IV of the HER2 protein, thereby blocking ligand-independent HER2 homodimerization within cells with high HER2 overexpression, and to a lesser extent also blocking the heterodimerization of HER2 with other family members (Cho et al., Nature 2003; 421: 756-760 and Wehrman et al., Proc Natl Acad Sci 2006; 103: 19063-19068). In cells with moderate HER2 expression levels, Trastuzumab has been found to inhibit the formation of HER2/EGFR heterodimers (Wehrman et al., Proc Natl Acad Sci 2006; 103: 19063-19068; and Schmitz et al., Exp Cell Res 2009; 315: 659-670). Trastuzumab mediates antibody-dependent cellular cytotoxicity (ADCC) and prevents ectodomain shedding, which would otherwise lead to the formation of truncated constitutively active proteins in HER2-overexpressing cells. Moreover, inhibition of the *in vitro* and *in vivo* proliferation of tumor cells expressing HER2 at high levels has also been reported for Trastuzumab (reviewed in Nahta and Esteva, Oncogene 2007; 26: 3637-3643). Trastuzumab has been approved by NMPA and FDA for the first-line therapy and adjuvant therapy of HER2-overexpressing metastatic breast cancers, whether in combination with chemotherapy or as a single agent after one or more chemotherapies. It has been found to be effective only in 20%-50% of HER2-overexpressing breast cancer patients, and many initial responders showed recurrence a few months later (Dinh et al., Clin Adv Hematol Oncol 2007; 5: 707-717). The National Comprehensive Cancer Network (NCCN) in the 2019 clinical treatment guidelines for breast cancers lists Pertuzumab and Trastuzumab in combination with docetaxel (a first category of recommendation) or paclitaxel as the first-line medication regimen for HER2-positive advanced (metastatic) breast cancers and neoadjuvant therapy (i.e., pre-surgery), which is expected to increase the success rate of breast cancer surgery and reduce the recurrence rate, and improve the survival and quality of life.

Pertuzumab (Omnitarg^{™}) is another humanized monoclonal antibody. It targets domain II of the HER2 protein, resulting in inhibition of ligand-induced heterodimerization (i.e., the dimerization of HER2 with another member of the ErbB family that has bound a ligand); and it has been reported to not strictly require the mechanism of HER2 expression at high levels (Franklin et al., Cancer Cell 2004; 5: 317-328). Although Pertuzumab also mediates ADCC, the main mechanism of action of Pertuzumab relies on its blocking of dimerization (Hughes et al., Mol Cancer THER 2009; 8: 1885-1892). In addition, it has been found that Pertuzumab enhances EGFR internalization and down-regulation by inhibiting the formation of EGFR/HER2 heterodimers, which would otherwise restrain EGFRs on the surface of the plasma membrane (Hughes et al., Mol Cancer THER 2009; 8: 1885-1892). This is associated with the observed internalization of EGFR homodimers more efficiently than EGFR/HER2 dimers (Pedersen et al., Mol Cancer Res 2009; 7: 275-284). According to the latest clinical research results of CLEOPATRA, NeoSphere, VELVET and PERUSE, the combined application of Pertuzumab and Trastuzumab targeting different epitopes of HER2 may significantly improve the progression-free survival and median survival of HER2-positive breast cancer patients.

Another proposed HER2-based treatment method is to combine anti-HER2 antibodies against different epitopes of HER2, which combination has been reported to be more effective than anti-HER2 antibodies alone in reducing tumor growth in both *in vitro* and *in vivo* tumor models (Emde et al., Oncogene 2011; 30: 1631-1642; and Spiridon et al., Clin Cancer res 2002; 8: 1720-1730). For example, it has been reported that the complementary mechanisms of Pertuzumab and Trastuzumab lead to enhanced antitumor effects and efficacy when the two antibodies were combined in patients who showed progression during the prior therapy with Trastuzumab (Baselga et al., J Clin Oncol 2010; 28: 1138-1144). In a phase III clinical trial (CLEOPATRA), it has been shown that the antibody combination of Pertuzumab plus Trastuzumab together with docetaxel resulted in prolonged progression-free survival compared to the combination of Trastuzumab and docetaxel in patients who had previously untreated HER2-positive metastatic breast cancers.

The combination of Pertuzumab and Trastuzumab is more effective than the single use. The single-agent response rate of Pertuzumab is only 3.4%, but the combined use of Pertuzumab and Trastuzumab has a response rate of up to 21.4%. Nevertheless, both Trastuzumab and Trastuzumab are only suitable for the treatment of breast cancers that are strongly positive for HER2 (the IHC test result is 3+), and for breast cancers that are weakly positive for HER2, neither Trastuzumab nor Trastuzumab has a satisfactory therapeutic effect. In recent years, cell therapy has made extraordinary achievements in the treatment of hematological tumors. Therefore, some scientists have developed therapies for HER2-positive breast cancers based on Trastuzumab. Unfortunately, the patients died of severe side effects on Day 5 after the CAR-T cell infusion (Morgan et al., The American Society of Gene & Cell Therapy 2010; 4: 843-851). Bispecific antibodies against different epitopes of HER2 are monoclonal antibody drugs used to recognize two different epitopes on the surface of HER2, which comprise two different antibody heavy chain variable regions and two identical light chain variable regions (CN105829347A; CN105820251A; and CN105980409A), and may specifically bind to domains II and IV on the surface of HER2. The purpose is to provide a bispecific antibody with both Trastuzumab activity and Pertuzumab activity, in order to replace the current combination therapy of two antibodies, i.e., Trastuzumab plus Pertuzumab used in clinical practice. However, antibodies disclosed in the prior art (CN105829347A; CN105820251A; and CN105980409A) have the characteristics of poor antigen binding activity, or poor stability and easy to form precipitates, and stability is a prerequisite for antibody druggability.

### Summary of the Invention

In view of the above-mentioned technical defects of low affinity as well as poor stability and easy to form precipitates in the prior art, the purpose of the present invention is to provide a bispecific antibody against different epitopes of HER2 and an application thereof. The inventors of the present application have found that by using the heavy chains of Pertuzumab and Trastuzumab, respectively, and optimizing the light chains of natural Trastuzumab as the common light chains, a bispecific antibody can be obtained, which has comparable affinities for targets on HER2, i.e., domains II and IV to those of wild-type Pertuzumab and Trastuzumab, respectively. Due to the characteristics of bispecificity and high affinity, the antibody of the present invention is likely to be also effective for breast cancers that are weakly positive for HER2. Moreover, it has been unexpectedly found that the antibody of the present invention has an enhanced inhibitory effect on the proliferation of a variety of HER2-expressing tumor cells.

In order to solve the above-mentioned technical problems, the technical solution of a first aspect of the present invention is to provide an anti-HER2 bispecific antibody comprising a first protein functional region and a second protein functional region for respectively recognizing extracellular domain IV and extracellular domain II of HER2, wherein the first protein functional region comprises a first heavy chain and a first light chain, and the second protein functional region comprises a second heavy chain and a second light chain, wherein the first heavy chain comprises a heavy chain variable region as shown in the amino acid sequence of SEQ ID NO: 4 or a mutant thereof, the second heavy chain comprises a heavy chain variable region as shown in the amino acid sequence of SEQ ID NO: 3 or a mutant thereof, and the light chains comprise a light chain variable region comprising one or more amino acid substitutions at a position(s) selected from the following in the amino acid sequence set forth in SEQ ID NO: 1: R24, N30, T31, A32, F53, L54, S56, R66, H91, T93, T94 and P96. Here, the bispecific antibody may have an scFv, F(ab)2 or IgG-like structure.

In a particular preferred embodiment, in the light chain variable region of the bispecific antibody, the amino acid substitution(s) is/are to substitute the original amino acid residue with an amino acid residue selected from the group consisting of: Asp (D), Glu (E), Ser (S), Thr (T), Asn (N), Gln (Q), Gly (G), Ala (A), Val (V), Ile (I), Leu (L), Lys (K), Met (M), Phe (F), Tyr (Y), Trp (W) and Arg (R).

In a particular preferred embodiment, the light chain variable region of the bispecific antibody comprises one or more of the following amino acid residue substitutions in the amino acid sequence set forth in SEQ ID NO:1: R24K, N30S, T31I, A32G, F53Y, L54R, S56T, R66G, H91Y, T93I, T94Y and P96Y. Preferably, the amino acid residue substitution(s) is/are at one or more of the following positions: N30S, F53Y, L54R, S56T and P96Y.

In a particular preferred embodiment, the light chain variable region of the bispecific antibody comprises one or more amino acid residue substitutions in the amino acid sequence set forth in SEQ ID NO: 1 selected from the group consisting of: (1) F53Y; (2) F53Y, L54R and S56T; (3) P96Y; (4) N30S and F53Y; (5) N30S, F53Y, L54R and S56T; or (6) N30S and P96Y.

In a particular preferred embodiment, the bispecific antibody further comprises a light chain constant region, preferably a human lambda or kappa light chain constant region. Preferably, the light chain comprises one or more amino acid residue substitutions in the amino acid sequence set forth in SEQ ID NO: 2 selected from the group consisting of: (1) F53Y; (2) F53Y, L54R and S56T; (3) P96Y; (4) N30S and F53Y; (5) N30S, F53Y, L54R and S56T; or (6) N30S and P96Y.

In a particular preferred embodiment, the first heavy chain and the second heavy chain of the bispecific antibody further comprise a heavy chain constant region, preferably a human heavy chain constant region. Preferably, the first heavy chain and the second heavy chain of the bispecific antibody form a heterodimer preferably through linking via a "Knob-in-Hole" structure. More preferably, the heavy chain variable region of the second heavy chain comprises mutation D102E in the amino acid sequence as set forth in SEQ ID NO: 3.

In a particular preferred embodiment, the heavy chain variable region of the first heavy chain of the bispecific antibody comprises the following CDR sequences: HCDR1 as shown in SEQ ID NO: 5, HCDR2 as shown in SEQ ID NOs: 6-13, and HCDR3 as shown in SEQ ID NOs: 14-19;

Preferably, the heavy chain variable region of the first heavy chain comprises HCDR1 as shown in SEQ ID NO: 5, HCDR2 as shown in SEQ ID NO: 6, and HCDR3 as shown in SEQ ID NO: 15; or HCDR1 as shown in SEQ ID NO: 5, HCDR2 as shown in SEQ ID NO: 7, and HCDR3 as shown in SEQ ID NO: 16; or HCDR1 as shown in SEQ ID NO: 5, HCDR2 as shown in SEQ ID NO: 8, and HCDR3 as shown in SEQ ID NO: 15; or HCDR1 as shown in SEQ ID NO: 5, HCDR2 as shown in SEQ ID NO: 9, and HCDR3 as shown in SEQ ID NO: 15; or HCDR1 as shown in SEQ ID NO: 5, HCDR2 as shown in SEQ ID NO: 10, and HCDR3 as shown in SEQ ID NO: 15; or HCDR1 as shown in SEQ ID NO: 5, HCDR2 as shown in SEQ ID NO: 7, and HCDR3 as shown in SEQ ID NO: 17; or HCDR1 as shown in SEQ ID NO: 5, HCDR2 as shown in SEQ ID NO: 11, and HCDR3 as shown in SEQ ID NO: 17; or HCDR1 as shown in SEQ ID NO: 5, HCDR2 as shown in SEQ ID NO: 12, and HCDR3 as shown in SEQ ID NO: 17; or HCDR1 as shown in SEQ ID NO: 5, HCDR2 as shown in SEQ ID NO: 13, and HCDR3 as shown in SEQ ID NO: 17; or HCDR1 as shown in SEQ ID NO: 5, HCDR2 as shown in SEQ ID NO: 7, and HCDR3 as shown in SEQ ID NO: 18; or HCDR1 as shown in SEQ ID NO: 5, HCDR2 as shown in SEQ ID NO: 7, and HCDR3 as shown in SEQ ID NO: 19.

In a particular preferred embodiment, the bispecific antibody has a reduced fucose modification; and preferably, the antibody is free of fucose modification.

In order to solve the above-mentioned technical problems, the technical solution of a second aspect of the present invention is to provide a nucleic acid sequence encoding the bispecific antibody or an antigen-binding portion thereof as described above.

In order to solve the above-mentioned technical problems, the technical solution of a third aspect of the present invention is to provide an expression vector comprising the nucleic acid sequence of the second aspect above.

In order to solve the above-mentioned technical problems, the technical solution of a fourth aspect of the present invention is to provide a prokaryotic or eukaryotic cell comprising the expression vector of the third aspect above.

In order to solve the above-mentioned technical problems, the technical solution of a fifth aspect of the present invention is to provide a light chain comprising a light chain variable region, wherein the light chain variable region comprises one or more amino acid residue substitutions in the amino acid sequence set forth in SEQ ID NO: 1 selected from the group consisting of: (1) F53Y; (2) F53Y, L54R and S56T; (3) P96Y; (4) N30S and F53Y; (5) N30S, F53Y, L54R and S56T; or (6) N30S and P96Y. Preferably, the light chain further comprises a light chain constant region, preferably a human lambda or kappa light chain constant region. More preferably, the light chain comprises one or more amino acid residue substitutions in the amino acid sequence set forth in SEQ ID NO: 2 selected from the group consisting of: (1) F53Y; (2) F53Y, L54R and S56T; (3) P96Y; (4) N30S and F53Y; (5) N30S, F53Y, L54R and S56T; or (6) N30S and P96Y.

In order to solve the above-mentioned technical problems, the technical solution of a sixth aspect of the present invention is to provide an application of the light chain of the fifth aspect above in the preparation of an antibody; and preferably, the antibody is scFv, Fab', F(ab)2 or a full-length antibody, or a monoclonal antibody, a bispecific antibody or a multispecific antibody.

In order to solve the above-mentioned technical problems, the technical solution of a seventh aspect of the present invention is to provide an anti-HER2 bispecific antibody-drug conjugate, prepared by covalently coupling the bispecific antibody as described above to a cytotoxic drug. Preferably, the cytotoxic drug is a tubulin inhibitor, a topoisomerase inhibitor or a DNA minor groove inhibitor. More preferably, the tubulin inhibitor is selected from maytansines, or auristatin derivatives, or tubulin; the topoisomerase inhibitor is selected from DXd or derivatives thereof, or SN-38, or doxorubicin and derivatives thereof; and the DNA minor groove inhibitor is selected from calicheamicin, duocarmycin and derivatives thereof, pyrrolobenzodiazepines or indolinobenzodiazepines.

In order to solve the above-mentioned technical problems, the technical solution of an eighth aspect of the present invention is to provide a pharmaceutical composition comprising the bispecific antibody as described above. Preferably, the pharmaceutical composition further comprises a hyaluronidase. More preferably, the hyaluronidase is rHuPH20 or modified rHuPH20. Most preferably, it further comprises a pharmaceutically acceptable carrier or excipient.

In order to solve the above-mentioned technical problems, the technical solution of a ninth aspect of the present invention is to provide a method for preparing the bispecific antibody as described above, comprising the steps of:
(1) constructing the nucleotide sequences encoding the heavy chains and the nucleotide sequence encoding the light chains in the same vector or different vectors; and
(2) expressing the vector(s) constructed in the step above in different host cells or the same host cell.

In order to solve the above-mentioned technical problems, the technical solution of a tenth aspect of the present invention is to provide an application of the anti-HER2 bispecific antibody, the anti-HER2 bispecific antibody-drug conjugate and the pharmaceutical composition comprising the bispecific antibody as described above in the preparation of a medicament for treating HER2-expressing tumors. Preferably, the positive tumors comprise breast cancer, gastric cancer, prostate cancer, lung cancer, bladder cancer, ovarian cancer, colon cancer, esophageal cancer, and head and neck cancer, endometrial cancer, malignant melanoma, pharyngeal cancer, oral cancer and skin cancer; and more preferably, breast cancer, gastric cancer and lung cancer.

In addition, in order to solve the above-mentioned technical problems, the technical solution of an eleventh aspect of the present invention is to provide a kit combination comprising a kit A and a kit B; wherein the kit A comprises the anti-HER2 bispecific antibody, the anti-HER2 bispecific antibody-drug conjugate and the pharmaceutical composition comprising the bispecific antibody as described above; and the kit B comprises other antibodies and bispecific antibodies that target HER2 or other targets, genetically modified cells or pharmaceutical compositions. The kit A and the kit B are used in either order, which means that the kit A is used first and then the kit B is used, or vice versa.

The anti-HER2 bispecific antibody, the immunoconjugate and the pharmaceutical composition or the kit combination of the present invention may be administered to patients for the treatment of related tumors.

In the present invention, the HER2-recognizing antibody or an antigen-binding portion thereof or a mixture thereof or therapeutic immune cells comprising an antigen-binding portion thereof of the present invention may also be used in combination with chemotherapeutics and/or other antibodies. Therefore, the composition of the present invention may further comprise chemotherapeutics and/or other antibodies.

In the present invention, the chemotherapeutics include but are not limited to: doxorubicin (Adriamycin), cyclophosphamide and taxanes [paclitaxel (Taxol), and docetaxel (Taxotere)], capecitabine (Xeloda), gemcitabine (Gemzar), vinorelbine (Navelbine), tamoxifen, aromatase inhibitors (Arimidex, Femara and Aromasin), 5-FU plus leucovorin, irinotecan (camptosar), oxaliplatin, cisplatin, carboplatin, estramustine, mitoxantrone (Novantrone), prednisone, vincristine (Oncovin), etc., or a combination thereof.

On the basis of conforming to common knowledge in the art, the above-mentioned preferred conditions can be combined arbitrarily to obtain preferred embodiments of the present invention.

The reagents and raw materials used in the present invention are all commercially available.

The positive and progressive effects of the present invention are to obtain a bispecific monoclonal antibody with high affinity, good stability, strong tumor cell killing activity and high ADCC activity by mutating designated amino acids in the light chain variable region of Trastuzumab, the heavy chain variable region of Pertuzumab, and the heavy chain variable region of Trastuzumab.

### Brief Description of the Drawings

Figure 1 shows the results of the inhibitory activity of a bispecific antibody against different epitopes of human HER2 on the proliferation of MDA-MB-175 cells, in which A shows the results of KJ015w vs. Pertuzumab, and B shows the results of Trastuzumab vs. Pertuzumab.
Figure 2 shows the electrophoretograms of various bispecific monoclonal antibody proteins.
Figure 3 shows the results of the cell binding activities of various bispecific antibodies against different epitopes of human HER2.
Figure 4 shows the results of the internalization of various bispecific antibodies against different epitopes of human HER2 for NCI-N87 (A) and MCF-7 (B) cells.
Figure 5 shows the results of the inhibitory activities of various bispecific antibodies against different epitopes of human HER2 on the proliferation of MDA-MB-175 cells, in which A shows the results of KJ015-A, B shows the results of KJ015-B, C shows the results of KJ015-C, and D shows the results of KJ015-D.
Figure 6 shows the results of the inhibitory activities of various bispecific antibodies against different epitopes of human HER2 on the proliferation of BT474 cells, in which A shows the results of KJ015-A, B shows the results of KJ015-B and KJ015-C, and C shows the results of KJ015-D.
Figure 7 shows the results of the inhibitory activities of various bispecific antibodies against different epitopes of human HER2 on the proliferation of SK-BR-3 cells, in which A shows the results of KJ015-A, B shows the results of KJ015-B and KJ015-C, and C shows the results of KJ015-D.
Figure 8 shows the results of the inhibitory activities of various bispecific antibodies against different epitopes of human HER2 on the proliferation of NCI-N87 cells, in which A shows the results of KJ015-A and KJ015-B, and B shows the results of KJ015-C and KJ015-D.
Figure 9 shows the results of the inhibitory activities of various bispecific antibodies against different epitopes of human HER2 on the proliferation of Calu-3 cells, in which A shows the results of KJ015-A and KJ015-C, B shows the results of KJ015-B, and C shows the results of KJ015-D.
Figure 10 shows the results of the ADCC activities of various bispecific antibodies against different epitopes of human HER2 on JIMT-1 cells, in which A shows the results of KJ015-A and KJ015-B, and B shows the results of KJ015-C and KJ015-D.
Figure 11 shows the results of the ADCC activities of various bispecific antibodies against different epitopes of human HER2 on SK-BR-3 cells, in which A shows the results of KJ015-A and KJ015-B, and B shows the results of KJ015-C and KJ015-D.
Figure 12 shows the results of the ADCC activities of various bispecific antibodies against different epitopes of human HER2 on NCI-N87 cells, in which A shows the results of KJ015-A and KJ015-B, B shows the results of KJ015-C and KJ015-D, and C shows the results of KJ015-E.
Figure 13 shows the results of the ADCC activities of various bispecific antibodies against different epitopes of human HER2 on MDA-MB-175 cells, in which A shows the results of KJ015-A-KJ015-C, and B shows the results of KJ015-D and KJ015-E.

### Detailed Description of Embodiments

The technical solutions of the present invention will be further explained below through specific examples. It is to be understood by those skilled in the art that the examples are provided to merely aid the understanding of the present invention, and are not to be regarded as a specific limitation to the present invention.

In the following examples, the experimental methods without specific conditions were selected in accordance with conventional methods and conditions, or in accordance with the product instructions.

### Example 1: Construction and expression of bispecific antibody against different epitopes of human HER2.

DNA fragments encoding the heavy chains of Trastuzumab (comprising a "knob" structure), the heavy chains of Pertuzumab (comprising a "hole" structure) and the light chains of Trastuzumab (SEQ ID NO: 2) were synthesized by full gene synthesis, and cloned into the expression vectors constructed in house, respectively, which expression vectors were composed of the following elements:
1) glutamine synthetase gene as a selection marker; or neomycin resistance gene as a heavy chain selection marker;
2) origin of replication: ori;
3) origin of replication from the vector pUC18, which allowed such plasmids to replicate in *E. coli;*
4) β-lactamase gene, which conferred ampicillin resistance in *E. coli;*
5) immediate early enhancer and promoter from human cytomegalovirus; and
6) human 1-immunoglobulin polyadenylation ("poly A") signal sequence; and

As described above, immunoglobulin genes comprising the heavy or light chains were prepared by gene synthesis, and cloned into the pUC57 (Amp) plasmids. The pUC57 (Amp) plasmids comprising the heavy chain genes, the pUC57 (Amp) plasmids comprising the light chain genes and the corresponding expression plasmids were digested with enzymes HindIII and EcoRI, and the heavy chains and the light chains were directionally cloned into the corresponding plasmids, respectively. The procedures of digestion and ligation were carried out in accordance with the instructions of the commercially provided kit.

The constructed Trastuzumab heavy chain, Pertuzumab heavy chain and light chain expression vectors above were transformed into *E. coli* DH5α, respectively, and positive clones were picked and inoculated in 500 ml LB medium for expansion. DNAs were extracted and purified with the Ultrapure Plasmid DNA Purification Kit (Qiagen) in accordance with the manufacturer's instructions. The above plasmid DNAs comprising the heavy chain- and light chain-encoding sequences were co-transfected into CHO-K1 (Chinese hamster ovary cells, purchased from ATCC) at a ratio of 1 : 1 : 2-1 : 1 : 4 with the Lipofectin Kit (Invitrogen), and the procedures were carried out in accordance with the manufacturer's instructions.

24-48 hours after transfection, the cell culture medium was replaced with a selection medium containing selection drugs, and the selection medium was replaced every 3-4 days until cell clones were formed. When the diameter of the cell clones reached 1-2 mm, single clones were picked from the plate with a cloning ring , and transferred to a 24-well plate. When single-cell clones grow to 50%-70% confluence in the 24-well plate, the culture supernatant of each clone was subjected to ELISA detection, and cells with high expression and good growth were selected for expansion and culture.

The above-mentioned ELISA detection was carried out as follows: the Trastuzumab-specific antigen (Rhinogen^{®}) and the Pertuzumab-specific antigen (Rhinogen^{®}) were diluted to a certain concentration (2 µg/ml) with a coating solution (pH 9.6 CBS), respectively, and then were used to coat a 96-well plate at 100 µL/well, and placed at 2°C-8°C overnight. The liquid in the wells was discarded, the plate was washed 3 times with PBST, a blocking solution (1% BSA PBST) was added at 300 (µL/well for 2 hr at room temperature after the plate was spin-dried, and the plate was washed 3 times with PBST and spin-dried again. The standards Trastuzumab and Pertuzumab were diluted into 7 concentration points with diluent PBS (pH 7.0) starting from 100 ng/mL by serial twofold dilutions. The expression supernatants were diluted as appropriate, and added to a 96-well plate at 100 (µL/well in duplicate, the plate was incubated at 37°C for 1 hr, the liquid was discarded, and the plate was washed 3 times and spin-dried. The enzyme-conjugated antibody (HRP-anti-human IgGy light chain specific antibody, Thermo) was diluted with a diluent to a certain concentration (1 : 2000), the dilution was added to the 96-well plate at 100 µL/well, the mixture was allowed to react at 37°C for 1 hr, the liquid was discarded, and the plate was washed 3-6 times and spin-dried. A substrate mixture was prepared and added to the 96-well plate at 100 µL/well, and the plate was incubated at 37°C for 20 min. A stop solution was added at 50 µL/well to stop the reaction. The absorbance values OD were read at 490 nm, and the contents of the samples were calculated according to the standard curve.

Finally, wild-type KJ015w antibody-expressing cells were obtained. Each cell was expanded to a volume of 100 ml with CD2 basal medium (Rhinogen^{®}) for fed-batch culture. Starting from Day 3,2% Feed4 (Rhinogen^{®}) was supplemented every day. After 12 days of suspension culture, the culture supernatants were collected, respectively, and purified by Protein A affinity chromatography to obtain a bispecific monoclonal antibody protein sample.

### Example 2: Determination of affinity of bispecific antibody against different epitopes of human HER2 for HER2.

The affinities of the single-target anti-HER2 antibodies Trastuzumab and Pertuzumab as well as the anti-HER2 bispecific antibody KJ015w for three forms of HER2 antigen were determined with Biacore T200.

First, the anti-human Fc (AHC) antibody was coupled to the CM5 chip, then the antibody IgG (2 µg/ml) to be tested was captured by AHC, and subsequently, three antigen molecules of different concentrations were flowed over the surface of the chip where the IgG was captured. The binding activities of the anti-HER2 antibody to the antigens of different concentrations were determined, and the obtained data were fitted according to the Biacore T200 analysis software to obtain the exact kinetic constants. The comparison results are shown in Table 2.

**Table 2: Determination results of affinities of antibody KJ015w for three forms of HER2 antigen.**

| **Antigen** | **Sample name** | **ka (1/Ms)** | **kd (1/s)** | **KD (M)** |
|---|---|---|---|---|
| HER2 extracellular domain full-length antigen (Rhinogen^{®}) | Trastuzumab | 3.86E+04 | 3.90E-05 | 1.01E-09 |
| | Pertuzumab | 2.23E+04 | 1.11E-04 | 4.98E-09 |
| | KJ015w | 4.71E+04 | 7.97E-05 | 1.69E-09 |
| Trastuzumab-specific antigen (Rhinogen^{®}) | Trastuzumab | 1.74E+04 | 4.91E-05 | 2.83E-09 |
| | Pertuzumab | No binding | | |
| | KJ015w | 2.84E+04 | 9.75E-05 | 3.43E-09 |
| Pertuzumab-specific antigen (Rhinogen^{®}) | Trastuzumab | No binding | | |
| | Pertuzumab | 4.40E+04 | 1.24E-04 | 2.81E-09 |
| | KJ015w | 1.16E+04 | 3.09E-03 | 2.67E-07 |

Trastuzumab, Pertuzumab and the anti-HER2 bispecific antibody KJ015w could all bind to the HER2 extracellular domain full-length antigen, and the anti-HER2 bispecific antibody KJ015w could recognize both the Pertuzumab-specific HER2 antigen (Rhinogen^{®}) and the Trastuzumab-specific HER2 antigen (Rhinogen^{®}). However, its affinity for the Pertuzumab-specific HER2 antigen was 2 orders of magnitude lower than that for the Pertuzumab-specific HER2 antigen.

### Example 3: Determination of inhibitory activity of bispecific antibody against different epitopes of human HER2 on cell proliferation.

The MDA-MB-175 cell culture flask was placed in a 37°C, 5% carbon dioxide constant temperature incubator for static culture. The color of the medium and the cell confluence were observed every day, and the cell doubling time was recorded. The cells were subcultured every 2-4 days. When the cells reached about 80% confluence, the cells were digested and plated. The medium was discarded, and the cells were washed once with PBS and digested with 0.25% trypsin (Gibco). The cells were collected by pipetting into a centrifuge tube, and the centrifuge tube was centrifuged at 500g for 3 min. The supernatant was discarded, a complete medium was added to resuspend the cells, and 100 µL of cell suspension was removed for counting. The cell density was adjusted to 1 × 10⁵ cells/mL with a medium containing 2% FBS, and the cells were plated in a 96-well plate at 100 µL/well.

The drugs to be tested were diluted with a medium containing 2% FBS as follows: the drugs to be tested were diluted to 150 µg/mL first, and then diluted 8 times at a 4-fold gradient to obtain drugs with 9 concentration gradients, in which the last concentration point was the negative control. For different cells, the starting antibody concentrations were different. For details, see the starting concentration points in the figures showing the results. 100 µL of the drug dilution was added to the corresponding wells of the 96-well plate with the cells plated in duplicate for each concentration point. The 96-well plate was incubated for reaction in a carbon dioxide incubator for 96-120 hours.

After the incubation was completed, the CCK8 reagent (Rhinogen^{®}) was added to the 96-well plate at 20 µL/well, and the plate was shaken and mixed well on a microplate shaker. The OD_{450 nm} absorbance values were read with a microplate reader 0.5-6 hours after dyeing. The raw data were subjected to curve fitting analysis with the GraphPad software.

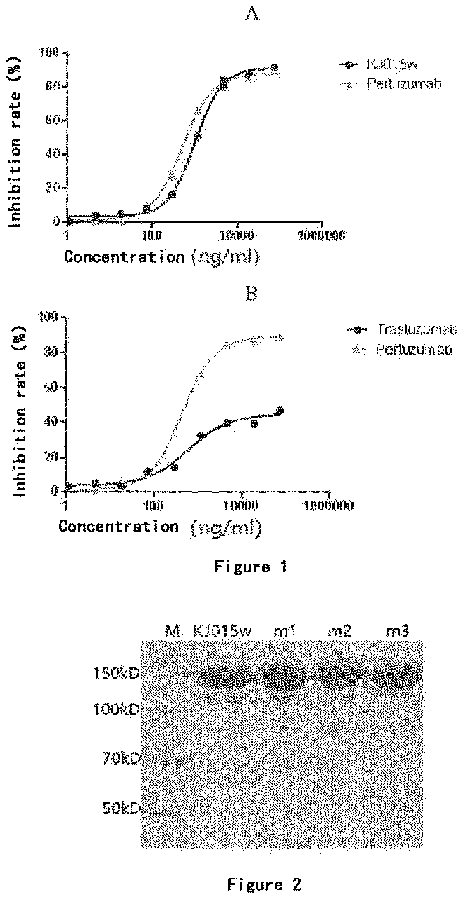

The results of the inhibitory activities of the antibodies on proliferation (Figure 1A) showed that KJ015w had an inhibitory activity on the proliferation of MDA-MB-175 cells.

### Example 4: Construction and expression of bispecific antibodies against different epitopes of human HER2.

DNA fragments encoding three light chain variants [respectively comprising one or more amino acid residue substitutions in the amino acid sequence set forth in SEQ ID NO: 2 selected from the group consisting of: F53Y (M1); F53Y, L54R and S56T (M2); and P96Y (M3)] were synthesized by full gene synthesis, and cloned into the antibody heavy and light chain expression vectors constructed in house, respectively.

The constructed Trastuzumab heavy chain, Pertuzumab heavy chain and light chain expression vectors above were transformed into *E. coli* DH5α, respectively, and positive clones were picked and inoculated in 500 ml LB medium for expansion. DNAs were extracted and purified with the Ultrapure Plasmid DNA Purification Kit (Qiagen) in accordance with the manufacturer's instructions. The above plasmid DNAs comprising the heavy chain- and light chain-encoding sequences were co-transfected into CHO-K1 at a certain ratio (1 : 1 : 2) with the Lipofectin Kit (Invitrogen), and the procedures were carried out in accordance with the manufacturer's instructions.

24-48 hours after transfection, the cell culture medium was replaced with a selection medium containing selection drugs, and the selection medium was replaced every 3-4 days until cell clones were formed. When the diameter of the cell clones reached 1-2 mm, single clones were picked from the plate with a cloning ring , and transferred to a 24-well plate. When single-cell clones grow to 50%-70% confluence in the 24-well plate, the culture supernatant of each clone was subjected to ELISA detection, and cells with high expression and good growth were selected for expansion and culture.

The above-mentioned ELISA detection was carried out as follows: the Trastuzumab-specific antigen (Rhinogen^{®}) and the Pertuzumab-specific antigen (Rhinogen^{®}) were diluted to a certain concentration (2 µg/ml) with a coating solution (pH 9.6 CBS), respectively, and then were used to coat a 96-well plate at 100 µL/well, and placed at 2°C-8°C overnight. The liquid in the wells was discarded, the plate was washed 3 times with PBST, a blocking solution (1% BSA PBST) was added at 300 µL/well for 2 hr at room temperature after the plate was spin-dried, and the plate was washed 3 times with PBST and spin-dried again. The standards Trastuzumab and Pertuzumab were diluted into 7 concentration points with diluent PBS (pH 7.0) starting from 100 ng/mL by serial twofold dilutions. The expression supernatants were diluted as appropriate, and added to a 96-well plate at 100 µL/well in duplicate, the plate was incubated at 37°C for 1 hr, the liquid was discarded, and the plate was washed 3 times and spin-dried. The enzyme-conjugated antibody (HRP-anti-human IgGy light chain specific antibody, Thermo) was diluted with a diluent to a certain concentration (1 : 2000), the dilution was added to the 96-well plate at 100 µL/well, the mixture was allowed to react at 37°C for 1 hr, the liquid was discarded, and the plate was washed 3-6 times and spin-dried. A substrate mixture was prepared and added to the 96-well plate at 100 µL/well, and the plate was incubated at 37°C for 20 min. A stop solution was added at 50 µL/well to stop the reaction. The absorbance values OD were read at 490 nm, and the contents of the samples were calculated according to the standard curve.

Finally, wild-type mutant KJ015ml, KJ015m2 and KJ015m3 antibody-expressing cells were obtained. Each cell was expanded to a volume of 100 ml with CD2 basal medium (Rhinogen^{®}) for fed-batch culture. Starting from Day 3, 2% Feed4 (Rhinogen^{®}) was supplemented every day. After 12 days of suspension culture, the culture supernatants were collected, respectively, and initially purified by Protein A affinity chromatography to obtain 3 bispecific monoclonal antibody protein samples comprising common light chains (Figure 2). Figure 2 showed that the purities of the 3 bispecific monoclonal antibody proteins were all high.

### Example 5: Determination of affinities of bispecific antibodies against different epitopes of human HER2 for HER2.

The Trastuzumab-specific antigen (Rhinogen^{®}), the Pertuzumab-specific antigen (Rhinogen^{®}) and the full-length HER2 antigen (Rhinogen^{®}) were diluted to a certain concentration (2 µg/ml) with a coating solution (pH 9.6 CBS), respectively, and then were used to coat a 96-well plate at 100 µL/well, and placed at 2°C-8°C overnight. The liquid in the wells was discarded, the plate was washed 3 times with PBST, a blocking solution (1% BSA PBST) was added at 300 µL/well for 2 hr at room temperature after the plate was spin-dried, and the plate was washed 3 times with PBST and spin-dried again. The standards Trastuzumab and Pertuzumab were diluted into 10 gradients with diluent PBS (pH 7.0) starting from 2000 ng/mL by serial three-fold dilutions. The dilutions were added to a 96-well plate at 100 µL/well in duplicate, and the plate was incubated at 37°C for 1 hr. The liquid was discarded, and the plate was washed 3 times and spin-dried. The enzyme-conjugated antibody (HRP-anti-human IgGy light chain specific antibody, Thermo) was diluted with a diluent to a certain concentration (1 : 2000), the dilution was added to the 96-well plate at 100 µL/well, the mixture was allowed to react at 37°C for 1 hr, the liquid was discarded, and the plate was washed 3-6 times and spin-dried. A substrate mixture was prepared and added to the 96-well plate at 100 µl/well, and the plate was incubated at 37°C for 20 min. A stop solution was added at 50 µL/well to stop the reaction.

**Table 3: Determination results of affinities of each anti-HER2 antibody for different epitope antigens of HER2 by ELISA.**

| Antibody name | EC₅₀ for Pertuzumab-specific antigen (ng/mL) | EC₅₀ for Trastuzumab-specific antigen (ng/mL) | EC₅₀ for full-length HER2 antigen (ng/mL) |
|---|---|---|---|
| Trastuzumab | / | 53.6 | 23.3 |
| Pertuzumab | 15.3 | / | 29.5 |
| KJ015w | 352.3 | 53.5 | 26.5 |
| KJ015ml | 257.2^{∗∗∗} | 38^{∗∗} | 25.5 |
| KJ015m2 | **125.1***** | 48.6 | 31.2 |
| KJ015m3 | 211.7^{∗∗∗} | 46.3 | 15.1*** |

| | | | |
|---|---|---|---|
| Note: ^{∗∗} P < 0.01 vs KJ015w; and ^{∗∗∗} P < 0.001 vs KJ015w. | | | |

The results in Table 3 showed that all the bispecific antibodies KJ015m1, KJ015m2 and KJ015m3 of the present invention had higher binding affinities for the Pertuzumab-specific antigen than the wild-type (i.e., KJ015w) (P < 0.001). The binding of the bispecific antibodies of the present invention to the Trastuzumab-specific antigen was comparable to that of the wild-type, in which the affinity of KJ015m1 was slightly higher than that of the wild-type (P < 0.01). In the binding experiment with the full-length HER2 antigen, KJ015m1 and KJ015m2 performed comparably to the wild-type, and the affinity of KJ015m3 was significantly higher than that of the wild-type (P < 0.001).

### Example 6: Determination of thermal stabilities of bispecific antibodies against different epitopes of human HER2.

The antibodies to be tested were diluted to 1 mg/ml with PBS, and the SYPRO orange dye (Life technologies) was diluted to 40× with deionized water. To 12.5 µL of sample dilution, 2.5 µl of 40× dye and 5 µL of deionized water were added. The mixture was added to a plate at 20 µL/well in duplicate. The plate was loaded into an instrument after being sealed, and the reaction was performed with a PCR reaction program of 25°C running for 2 minutes and 95°C running for 2 minutes. The raw readouts were exported, and the results were imported into Graphpad Prism for curve fitting analysis. The Tm values of each sample obtained are shown in Table 4, and the results showed that all the light chain variants M1-M3 had good thermal stabilities.

**Table 4: Thermal stability data of bispecific antibodies against different epitopes of human HER2.**

| **Sample name** | **Tm**_{Onset} **(°C)** | **Tm₁ (°C)** | **Tm₂ (°C)** |
|---|---|---|---|
| **KJ015w** | 65.4 | 71.7 | 87.0 |
| **KJ015m1** | 63.4 | 71.2 | 85.7 |
| **KJ015m2** | 65.1 | 70.9 | 86.5 |
| **KJ015m3** | 64.7 | 71.0 | 83.2 |

### Example 7: Heavy chain modification of bispecific antibodies against different epitopes of human HER2.

The light chain variants M1-M3 were selected as the light chains, and the heavy chains of Pertuzumab were mutated. Using the mixed light chains of M1, M2 and M3, the target region of a single heavy chain template was subjected to high-throughput mutations by employing the Kunkel technology, and two libraries were constructed: the mutation regions of library I were in HCDR1, HCDR2 and HCDR3; and the mutation region of library II was in HCDR2. The supercompetent cells were subjected to electroporation, respectively, and cultured for expansion. Phage library I and library II were harvested, respectively.

Using the antigen recombinant protein HER2 ECDs (Rhinogen^{®}) as baits, based on the solid-phase screening of maxi-sorp 96-well plate, the titer test was used to verify whether the phages were enriched. Library I and library II were used for screening, respectively. For the phages enriched respectively in each library, 48 single clones were picked out for culture. The cultured phages were collected for ELISA identification. The positive single clones with higher affinity than the wild-type antibody were picked out, and subjected to DNA sequencing to obtain the antibody sequence.

### Example 8: Determination of affinity constants of candidate sequences.

Based on the plasmids of Example 1, the plasmid DNAs of the heavy chain- and light chain-encoding sequences were co-transfected into ExpiCHO-S cells (Gibco) at a certain ratio (1 : 1), and the procedures were carried out in accordance with the instructions of the Transfection Kit (Rhinogen^{®}). The cells were cultured for 7-10 days after being transfected, and the culture supernatants were collected and purified by Protein A affinity chromatography for determination of the affinity constants.

The affinities of single-target anti-HER2 antibodies for the HER2 ECD antigen (Rhinogen^{®}) were determined with Biacore T200. First, the anti-human Fc (AHC) antibody was coupled to the CM5 chip, then the antibodies IgG (2 µg/ml) to be tested were captured by AHC, and subsequently, three antigen molecules of different concentrations were flowed over the surface of the chip where the IgGs were captured. The binding activities of the anti-HER2 antibodies to the antigens of different concentrations were determined, and the obtained data were fitted according to the Biacore T200 analysis software to obtain the exact kinetic constants. The detailed results of the affinity constants are shown in Table 5. According to the affinity constant data, the affinity constants of the affinity-modified heavy chains of Pertuzumab were both increased by two orders of magnitude compared with that of the wild-type heavy chains of Pertuzumab. Compared with the wild-type heavy chains of Trastuzumab, the affinity of the Trastuzumab heavy chain D102E was about 3 times higher.

**Table 5: Affinity constants of candidate sequences.**

| Light chain | Heavy chain No. | Heavy chain amino acid sequence No. | | ka (1/Ms) | kd (1/s) | KD (M) |
|---|---|---|---|---|---|---|
| | | HCDR2 | HCDR3 | | | |
| M1 | 1 | SEQ ID NO: 6 | SEQ ID NO: 15 | 6.26E+04 | 3.12E-04 | 4.99E-09 |
| M1 | 2 | SEQ ID NO: 7 | SEQ ID NO: 16 | 5.25E+04 | 2.61E-04 | 4.97E-09 |
| M2 | 3 | SEQ ID NO: 8 | SEQ ID NO: 15 | 5.93E+04 | 1.52E-04 | 2.57E-09 |
| M1 | 4 | SEQ ID NO: 9 | SEQ ID NO: 15 | 6.51E+04 | 1.99E-04 | 3.05E-09 |
| M1 | 5 | SEQ ID NO: 10 | SEQ ID NO: 15 | 7.10E+04 | 1.43E-04 | 2.02E-09 |
| N30S M1 | 1 | SEQ ID NO: 6 | SEQ ID NO: 15 | 5.62E+04 | 2.84E-04 | 5.05E-09 |
| N30S M1 | 2 | SEQ ID NO: 7 | SEQ ID NO: 16 | 5.39E+04 | 2.72E-04 | 5.04E-09 |
| N30S M2 | 3 | SEQ ID NO: 8 | SEQ ID NO: 15 | 5.80E+04 | 1.55E-04 | 2.68E-09 |
| N30S M1 | 4 | SEQ ID NO: 9 | SEQ ID NO: 15 | 6.36E+04 | 2.02E-04 | 3.18E-09 |
| N30S M1 | 5 | SEQ ID NO: 10 | SEQ ID NO: 15 | 6.96E+04 | 1.45E-04 | 2.09E-09 |
| M1 | 6 | SEQ ID NO: 6 | SEQ ID NO: 14 | 3.62E+04 | 1.81E-03 | 5.01E-08 |
| Pertuzumab light chain | 6 | SEQ ID NO: 6 | SEQ ID NO: 14 | 7.17E+04 | 1.23E-04 | 1.72E-09 |
| N30S M1 | 7 | Trastuzumab heavy chain | | 1.16E+05 | 1.05E-04 | 9.05E-10 |
| N30S M2 | 7 | Trastuzumab heavy chain | | 1.08E+05 | 9.87E-05 | 9.14E-10 |
| N30S M1 | 8 | Trastuzumab heavy chain D102E | | 1.20E+05 | 4.73E-05 | 3.95E-10 |
| N30S M2 | 8 | Trastuzumab heavy chain D102E | | 1.09E+05 | 4.18E-05 | 3.83E-10 |
| M1 | 7 | Trastuzumab heavy chain | | 1.12E+05 | 1.14E-04 | 1.02E-09 |
| M1 | 9 | SEQ ID NO: 11 | SEQ ID NO: 17 | 6.83E+04 | 3.86E-04 | 5.66E-09 |
| M1 | 10 | SEQ ID NO: 12 | SEQ ID NO: 17 | 6.79E+04 | 4.47E-04 | 6.58E-09 |
| M1 | 11 | SEQ ID NO: 13 | SEQ ID NO: 17 | 6.85E+04 | 3.93E-04 | 5.74E-09 |
| M1 | 12 | SEQ ID NO: 7 | SEQ ID NO: 17 | 7.54E+04 | 2.53E-04 | 3.35E-09 |
| M1 | 13 | SEQ ID NO: 7 | SEQ ID NO: 18 | 4.55E+04 | 4.09E-04 | 8.98E-09 |
| M1 | 14 | SEQ ID NO: 7 | SEQ ID NO: 19 | 6.47E+04 | 6.07E-04 | 9.38E-09 |

| | | | | | | |
|---|---|---|---|---|---|---|
| Note: Among the candidate heavy chains in Table 5, relative to the variable region sequences of the heavy chains of Pertuzumab, mutations were only in the HCDR2 and HCDR3 regions. In addition, all the sequences of HCDR1 were SEQ ID NO: 5. | | | | | | |

### Example 9: Preparation of bispecific antibodies against different epitopes of human HER2.

Based on the plasmids of Example 1, the genes of the antibody heavy chains were synthesized in accordance with Table 7, and the plasmids were extracted to obtain the corresponding heavy chain plasmids. The light chain N30S M1, the light chain N30S M2 and the Trastuzumab heavy chain D102E were constructed in accordance with the same procedures. The above plasmid DNAs comprising the heavy chain- and light chain-encoding sequences were co-transfected into ExpiCHO-S cells (Gibco) at a certain ratio (1 : 1 : 2) with the Transient Transfection Kit (Rhinogen^{®}), and the procedures were carried out in accordance with the instructions of the Rhinogen^{®} Transfection Kit. The cells were cultured for 7-10 days after being transfected, and the culture supernatants were collected and purified by Protein A affinity chromatography for functional evaluation. The purified samples were sterilized and filtered with a 0.22 µm membrane, and stored at 2°C-8°C. See Table 6 for the information about the samples obtained. During the samples were stored, it was found that only sample KJ015-E was precipitated, and all the other samples were clear, indicating that sample KJ015-E was unstable.

**Table 6: Sequence combinations of anti-HER2 bispecific antibodies.**

| Name | Common light chain | Second heavy chain | First heavy chain |
|---|---|---|---|
| KJ015-A | M1 | Trastuzumab heavy | Heavy chain No. 1 in |
| | | chain | Table 5 |
| KJ015-B | M1 | Trastuzumab heavy chain | Heavy chain No. 2 in Table 5 |
| KJ015-C | N30S M1 | Trastuzumab heavy chain D102E | Heavy chain No. 1 in Table 5 |
| KJ015-D | N30S M1 | Trastuzumab heavy chain D102E | Heavy chain No. 2 in Table 5 |
| KJ015-E# | SEQ ID NO: 54 | SEQ ID NO: 92 | SEQ ID NO: 64 |

| | | | |
|---|---|---|---|
| #: The SEQ ID NOs were those from patent US20170029529. | | | |

### Example 10: Determination of purities of bispecific antibodies against different epitopes of human HER2.

The purities of the KJ015 antibodies were determined by reference to size exclusion chromatography (Appendix III B, Part III, Chinese Pharmacopoeia (2010th Edition)). Hydrophilic modified silica gel was used as the filler (TSKgel SuperSW mAb HR, 300 × 7.8 mm); a mixed solution of disodium hydrogen phosphate and isopropanol (28.4 g of disodium hydrogen phosphate was dissolved with purified water, the solution was diluted to 800 ml, the pH value was adjusted to 7.0 with phosphoric acid, 10 ml of isopropanol was added, and the volume was adjusted to 1 L with purified water) was used as the mobile phase; the flow rate was 0.7 ml/min; and the detection wavelength was 280 nm. 20 µl of the test solution was injected into the liquid chromatograph for detection. The monomer peak areas of the antibodies were calculated in accordance with the peak area normalization method. The KJ015 samples were subjected to CE-SDS detection with the PA800 plus instrument by reference to the official documents of USP Medicines Compendium Bevacizumab and Beckman Coulter. The purity values of each sample obtained are shown in Table 7. It can be seen that the purity of each antibody was high and similar to those of Trastuzumab and Pertuzumab, and could be used for subsequent activity evaluation.

**Table 7: Purity of each antibody sample to be tested.**

| **Sample name** | **Purity (%)** | |
|---|---|---|
| | **SEC** | **CE** |
| KJ015-A | 98 | 96.135 |
| KJ015-B | 99.84 | 96.018 |
| KJ015-C | 93.80 | 96.088 |
| KJ015-D | 97.44 | 94.006 |
| KJ015-E | 97.50 | 94.065 |
| Trastuzumab | 99.76 | / |
| Pertuzumab | 99.61 | / |

### Example 11: Determination of thermal stabilities of bispecific antibodies against different epitopes of human HER2.

The antibodies to be tested were diluted to 1 mg/mL with PBS, and the SYPRO orange dye (Life technologies) was diluted to 40× with deionized water. To 12.5 µL of sample dilution, 2.5 µl of 40× dye and 5 µL of deionized water were added. The mixture was added to a plate at 20 µL/well in duplicate. The plate was loaded into an instrument after being sealed, and the reaction was performed with a PCR reaction program of 25°C running for 2 minutes and 95°C running for 2 minutes. The raw readouts were exported, and the results were imported into Graphpad Prism for curve fitting analysis. The Tm values of each sample obtained are shown in Table 8. It can be seen that there was little difference in the Tm value of each antibody, and their Tm values were similar to those of Trastuzumab and Pertuzumab.

**Table 8: Tm value of each sample.**

| **Sample name** | **Tm (°C)** |
|---|---|
| KJ015-A | 68.69 |
| KJ015-B | 67.76 |
| KJ015-C | 68.91 |
| KJ015-D | 68.78 |
| KJ015-E | 68.42 |
| Trastuzumab | 70.77 |
| Pertuzumab | 68.68 |

### Example 12: Determination of cell binding activities of bispecific antibodies against different epitopes of human HER2.

The cell culture flask was placed in a 37°C, 5% carbon dioxide constant temperature incubator for static culture. The color of the medium and the cell confluence were observed every day, and the cell doubling time was recorded. The cells were subcultured every 2-4 days. When the cells reached about 80% confluence, the cells were digested and plated. The medium was discarded, and the cells were washed once with PBS and digested with 0.25% trypsin (Gibco). The cells were collected by pipetting into a centrifuge tube, and the centrifuge tube was centrifuged at 500g for 3 min. The supernatant was discarded, a complete medium was added to resuspend the cells, and 100 µL of cell suspension was removed for counting. The cells were resuspended in PBS (containing 1% BSA), and the cell density was adjusted to 3×10⁶ cells/mL.

The drugs to be tested were diluted with a medium containing 2% FBS as follows: the drugs to be tested were diluted to a fixed concentration first, and then diluted 5 times at a 3-fold gradient to obtain drugs with 6 concentration gradients. For different cells, the starting antibody concentrations were different. For details, see the starting concentration points in the figures showing the results. To 100 µL of the resuspended cells, the antibodies to be tested were added at a final concentration of 300 nM-1 nM, the isotype IgG (Rhinogen^{®}) was used as a negative control, and the mixture was incubated at 4°C for 1 hour. After the incubation was completed, the incubate was washed 3 times with chilled PBS, the cells were resuspended with 50 µL of PBS (containing 1% BSA), the anti-human fluorescent secondary antibody was added, and the mixture was incubated at 4°C for half an hour. After the incubation was completed, the incubate was washed 3 times with chilled PBS, and loaded into an instrument for detection.

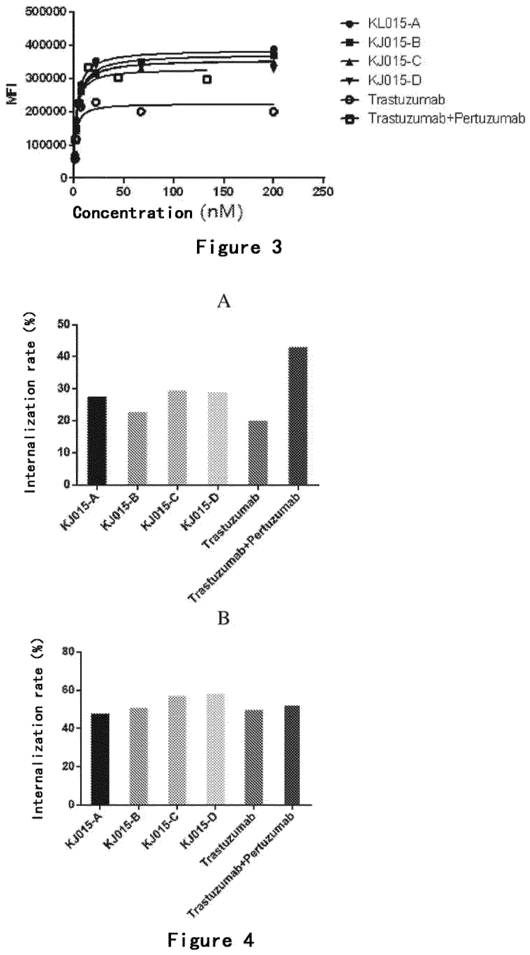

The detection results (Figure 3) showed that samples KJ015A-KJ015D all had binding activities, which were higher than that of Trastuzumab, and comparable to the results of the mixed sample of Tastuzumab + Pertuzumab.

### Example 13: Determination of internalization activities of bispecific antibodies against different epitopes of human HER2.

The cell culture flask was placed in a 37°C, 5% carbon dioxide constant temperature incubator for static culture. The color of the medium and the cell confluence were observed every day, and the cell doubling time was recorded. The cells were subcultured every 2-4 days. When the cells reached about 80% confluence, the cells were digested and plated. The medium was discarded, and the cells were washed once with PBS and digested with 0.25% trypsin (Gibco). The cells were collected by pipetting into a centrifuge tube, and the centrifuge tube was centrifuged at 500g for 3 min. The supernatant was discarded, a complete medium was added to resuspend the cells, and 100 µL of cell suspension was removed for counting. The cells were resuspended in PBS (containing 1% BSA), and the cell density was adjusted to 3×10⁶ cells/mL.

To 100 µL of the resuspended cells, the antibodies to be tested were added at a final concentration of 10 µg/mL (NCI-N87) or 50 µg/mL (MCF-7), the isotype IgG (Rhinogen^{®}) was used as a negative control, and the mixture was incubated at 4°C for 1 hour. The incubate was washed 3 times with chilled PBS, the cells were resuspended with 100 µL of complete medium, and the suspension was incubated in a 37°C cell incubator for 1 hour. After the incubation was completed, the incubate was washed 3 times with chilled PBS, the cells were resuspended with 50 µL of PBS (containing 1% BSA), the anti-human fluorescent secondary antibody was added, and the mixture was incubated at 4°C for half an hour. After the incubation was completed, the incubate was washed 3 times with chilled PBS, and loaded into an instrument for detection. Internalization (%) = 1 - (MFI of test sample-treated group at a certain time point - MFI of control hIgG at this time point) / (MFI of test sample-treated group at 0 hr - MFI of control hIgG at 0 hr) × 100%. The internalization rates of each antibody to be tested at each time point were calculated, and then plotted.

The internalization results are shown in Figure 4. For NCI-N87 cells, the internalization efficiencies of all 4 samples were between those of Trastuzumab and Trastuzumab + Pertuzumab. For MCF-7 cells, the activity of sample KJ015-A was slightly lower than those of Trastuzumab and Trastuzumab + Pertuzumab, the activity of sample KJ015-B was comparable to those these two antibodies, and the activities of samples KJ015-C and KJ015-D were higher than those of Trastuzumab and the combination of Trastuzumab + Pertuzumab.

### Example 14: Determination of inhibitory activities of bispecific antibodies against different epitopes of human HER2 on proliferation.

The method for inhibiting cell proliferation was used to evaluate the inhibition of the expression of HER2-positive tumor cells by different variants of anti-HER2 antibodies. The cell lines used were shown in Table 9.

The cell culture flask was placed in a 37°C, 5% carbon dioxide constant temperature incubator for static culture. The color of the medium and the cell confluence were observed every day, and the cell doubling time was recorded. The cells were subcultured every 2-4 days. When the cells reached about 80% confluence, the cells were digested and plated. The medium was discarded, and the cells were washed once with PBS and digested with 0.25% trypsin (Gibco). The cells were collected by pipetting into a centrifuge tube, and the centrifuge tube was centrifuged at 500g for 3 min. The supernatant was discarded, a complete medium was added to resuspend the cells, and 100 µL of cell suspension was removed for counting. The cell density was adjusted to 1×10⁵ cells/mL with a medium containing 2% FBS, and the cells were plated in a 96-well plate at 100 µL/well.

The drugs to be tested were diluted with a medium containing 2% FBS as follows: the drugs to be tested were diluted to 20-6000 µg/mL first, and then diluted 8-10 times at a 2.2-3-fold gradient to obtain drugs with 9-11 concentration gradients, in which the last concentration point was the negative control. For different cells, the starting antibody concentrations were different. For details, see the starting concentration points in the figures showing the results. 100 µL of the drug dilution was added to the corresponding wells of the 96-well plate with the cells plated in duplicate for each concentration point. The 96-well plate was incubated for reaction in a carbon dioxide incubator for 96-120 hours.

After the incubation was completed, the CCK8 reagent (Rhinogen^{®}) was added to the 96-well plate at 20 µL/well, and the plate was shaken and mixed well on a microplate shaker. The OD_{450 nm} absorbance values were read with a microplate reader 0.5-6 hours after dyeing. The raw data were subjected to curve fitting analysis with the GraphPad software.

**Table 9: Inhibition rate of cell proliferation.**

| **Sample name** | Maximum inhibition rate of cell proliferation (%) | | | | |
|---|---|---|---|---|---|
| | MDA-MB-175 | BT474 | SK-BR-3 | NCI-N87 | Calu-3 |
| Trastuzumab | 30.4 | 59.6 | 32.4 | 48.5±2.3 | 47.8 |
| Trastuzumab + Pertuzumab | 76.7±2.8 | 69.8±3.2 | 25.9±7.2 | 49.4±0.3 | 70.3±3.7 |
| KJ015-A | 75.7 | 83.1 | 56.4 | 79.6 | 81.2 |
| KJ015-B | 75.4 | 84.1 | 57.8 | 79.2 | 84.6 |
| KJ015-C | 81.5 | 84.8 | 58.2 | 82.5 | 82.0 |
| KJ015-D | 81.7 | 85.7 | 57.0 | 80.3 | 81.4 |

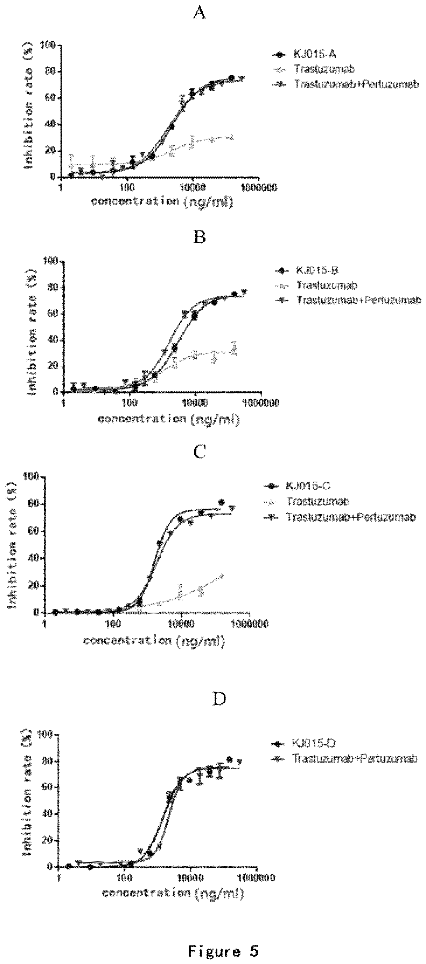

The results of the inhibitory activities on the proliferation of MDA-MB-175 cells (Figure 5) showed that all the samples A-D could inhibit the growth of MDA-MB-175 cells in a dose dependent manner, and the inhibitory activities on proliferation were significantly higher than that of Trastuzumab. The activities of samples KJ015-A and KJ015-B were comparable to that of the combination of Trastuzumab + Pertuzumab. The highest inhibition rates of samples KJ015-C and KJ015-D were 1.1 times that of the combination of Trastuzumab + Pertuzumab. The EC50 value of sample KJ015-D was 1.5 µg/ml, which was lower than 2.3 µg/ml of the combined sample of Trastuzumab + Pertuzumab.

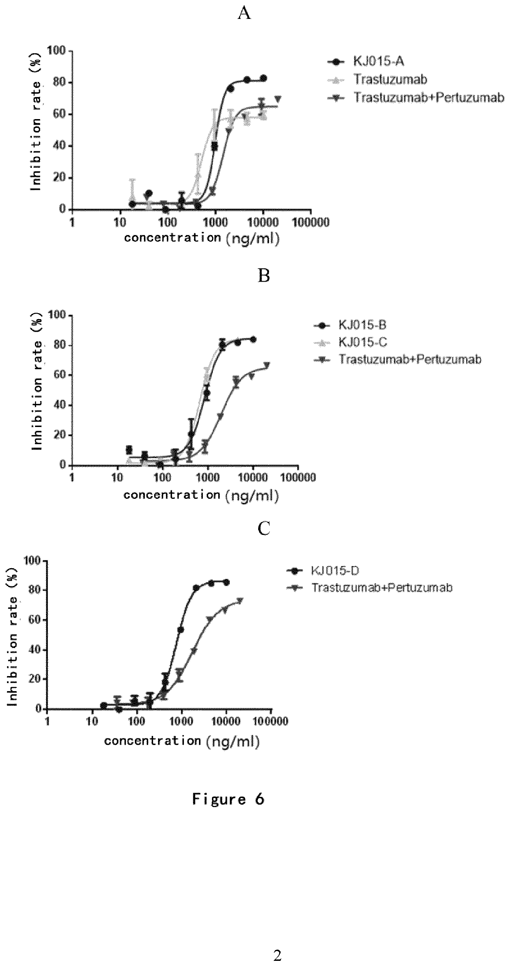

The results of the inhibitory activities on the proliferation of BT474 cells (Figure 6) showed that KJ015-A-KJ015-D could all inhibit the growth of BT474 cells in a dose dependent manner. The inhibitory activities on proliferation of the samples were significantly higher than those of Trastuzumab and the combination of Trastuzumab + Pertuzumab, and the maximum inhibition rates were all about 1.2 times that of the combination of Trastuzumab + Pertuzumab.

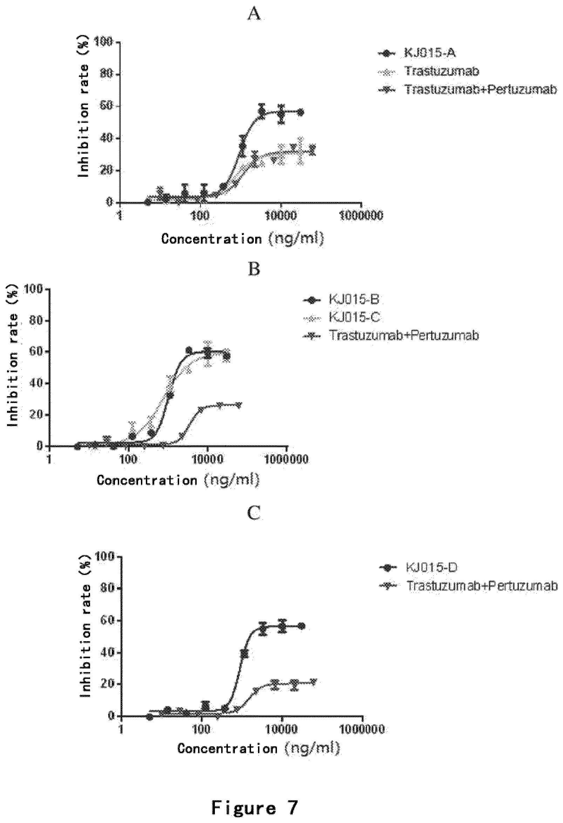

The results of the inhibitory activities on the proliferation of SK-BR-3 cells (Figure 7) showed that KJ015-A-KJ015-D could inhibit the growth of SK-BR-3 cells in a dose dependent manner. The inhibitory activities on proliferation of the samples were significantly higher than those of Trastuzumab and the combination of Trastuzumab + Pertuzumab, and the maximum inhibition rates were all about 2.2 times that of the combination of Trastuzumab + Pertuzumab.

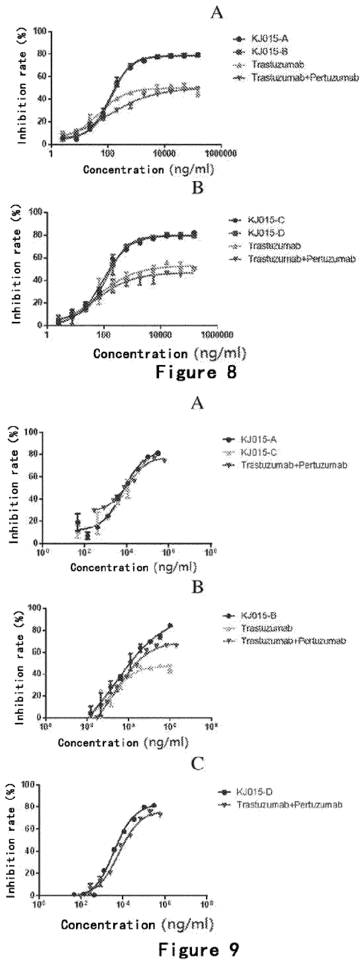

The results of the inhibitory activities on the proliferation of NCI-N87 cells (Figure 8) showed that the killing activities of 4 candidate antibodies on NCI-N87 cells were substantially comparable. The maximum killing rates for NCI-N87 cells were all higher than those of Trastuzumab and the combination of Trastuzumab + Pertuzumab, and the maximum inhibition rates were all about 1.6-1.7 times that of the combination of Trastuzumab + Pertuzumab.

The results of the inhibitory activities on the proliferation of Calu-3 cells (Figure 9) showed that KJ015-A-KJ015-D could all inhibit the growth of Calu-3 cells in a dose dependent manner. The inhibitory activities on proliferation of the samples were significantly higher than that of Trastuzumab, and were about 1.2 times that of the combination of Trastuzumab + Pertuzumab on average.

### Example 15: Determination of ADCC activities of bispecific antibodies against different epitopes of human HER2.

The cell culture flask was placed in a 37°C, 5% carbon dioxide constant temperature incubator for static culture. The color of the medium and the cell confluence were observed every day, and the cell doubling time was recorded. The cells were subcultured every 2-4 days. When the cells reached about 80% confluence, the cells were digested and plated. The medium was discarded, and the cells were washed once with PBS and digested with 0.25% trypsin (Gibco). The cells were collected by pipetting into a centrifuge tube, and the centrifuge tube was centrifuged at 500g for 3 min. The supernatant was discarded, a complete medium was added to resuspend the cells, and 100 µL of cell suspension was removed for counting. The cell density was adjusted to 2.5-7.5×10⁵ cells/mL with a medium containing 2% FBS, and the cells were plated in a 96-well plate at 100 µL/well.

After the 96-well plate was cultured overnight, the samples were added. The drugs to be tested were diluted with a medium containing 1% FBS as follows: the drugs to be tested were diluted to 75-300 µg/mL first (the start concentration could be adjusted according to different cells), and then diluted 8 times at a 4-5-fold gradient to obtain drugs with 9 concentration gradients, in which the last concentration point was the negative control. For different cells, the starting antibody concentrations were different. For details, see the starting concentration points in the figures showing the results. 25 µL of the drug dilution was added to the corresponding wells of the 96-well plate with the cells plated in duplicate for each concentration point. The effector cells Jurkat-NFAT/CD16a were diluted to 1-3×10⁶ cells/mL with 1640 + 1% FBS, and 25 uL of dilution was added to each well. (The number of effector cells could be adjusted according to different cells.) Enough sterile water was added to the edge of the 96-well plate, and then the 96-well plate was incubated for reaction in a carbon dioxide incubator for 4-6 hours.

After the incubation was completed, the cells to be tested were removed from the incubator and placed at room temperature (22°C-25°C) to equilibrate for at least 15 min, and then the luciferase reagent (Rhinogen^{®}) incubated at room temperature was added to the 96-well plate at 75 µL/well. The plate was shaken and mixed well on a microplate shaker, and placed at room temperature for 3-5 min. The fluorescence values were read with a microplate reader. The raw data were subjected to curve fitting analysis with the GraphPad software.

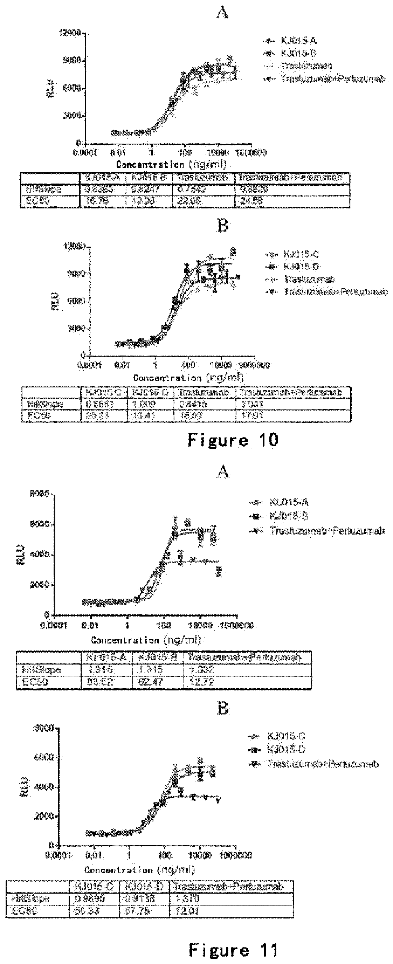

The results of the ADCC activities on JIMT-1 cells (Figure 10) showed that samples KJ015-A-KJ015-D all had ADCC activities in a dose dependent manner, in which the maximum signal values of sample A were 1.2 times those of Trastuzumab and the combination of Trastuzumab + Pertuzumab, respectively; for sample B, the maximum signal values were 1.3 times and 1.2 times those of Trastuzumab and the combination of Trastuzumab + Pertuzumab, respectively; for sample C, the maximum signal values were 1.5 times and 1.3 times those of Trastuzumab and the combination of Trastuzumab + Pertuzumab, respectively; and for sample D, the maximum signal values were 1.5 times and 1.3 times those of Trastuzumab and the combination of Trastuzumab + Pertuzumab, respectively; indicating that the ADCC activities of samples KJ015-A-KJ015-D were all higher than those of Trastuzumab and the combination of Trastuzumab + Pertuzumab.

The results of the ADCC activities on SK-BR-3 cells (Figure 11) showed that samples KJ015-A-KJ015-D all had ADCC activities in a dose dependent manner, in which the maximum signal values of the samples A-D were 1.8 times, 1.7 times, 1.7 times and 1.6 times that of the combination of Trastuzumab + Pertuzumab, respectively; indicating that the ADCC activities of samples KJ015-A-KJ015-D were all higher than that of the combination of Trastuzumab + Pertuzumab.

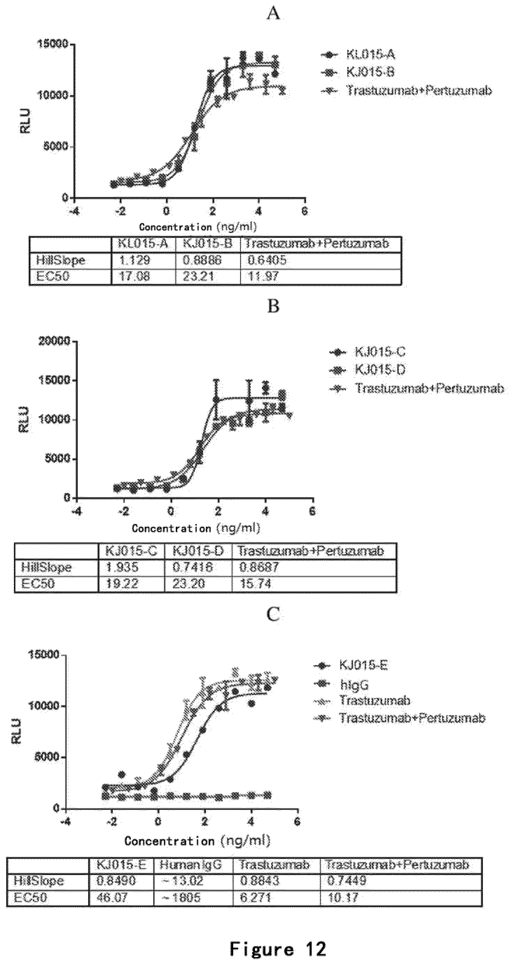

The results of the ADCC activities on NCI-N87 cells (Figure 12) showed that samples KJ015-A-KJ015-D all had ADCC activities in a dose dependent manner, in which the maximum signal values of the samples A-C were all about 1.2 times that of the combination of Trastuzumab + Pertuzumab; indicating that the ADCC activities of samples KJ015-A-KJ015-C were all higher than that of the combination of Trastuzumab + Pertuzumab. The ADCC activity of sample KJ015-D was comparable to that of the combination of Trastuzumab + Pertuzumab. The maximum activity signal value of sample KJ015-E was 0.9 times that of the combination of Trastuzumab + Pertuzumab, and the EC50 value was 46.07 ng/ml, which was significantly different from 10.17 ng/ml of the combination of Trastuzumab + Pertuzumab.

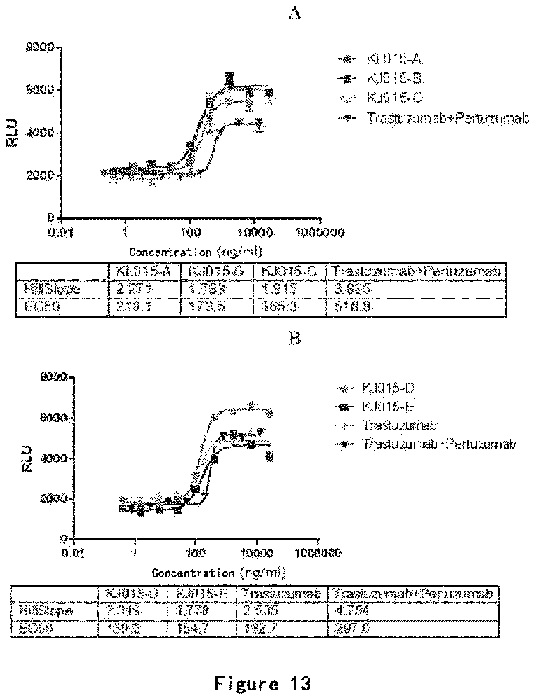

The results of the ADCC activities on MDA-MB-175 cells (Figure 13) showed that samples KJ015-A-KJ015-D all had ADCC activities in a dose dependent manner, in which the maximum signal values of the samples A-D were all about 1.2 times, 1.4 times, 1.3 times and 1.2 times that of the combination of Trastuzumab + Pertuzumab; indicating that the ADCC activities of samples KJ015-A-KJ015-D were all higher than that of the combination of Trastuzumab + Pertuzumab. The ADCC activity of sample KJ015-E was comparable to that of the combination of Trastuzumab + Pertuzumab.

The main sequences involved in the present invention are shown in Table 10 below.

**Table 10: Antibody sequence of the present invention.**

| SEQ ID NO | Description | Sequence |
|---|---|---|
| 1 | Trastuzumab light chain variable region WT | |
| 2 | Trastuzumab Light Chain WT | DIQMTQSPSSLSASVGDRVTITCRASQDVNT AVAWYQQKPGKAPKLLIYSASFLYSGVPSRF SGSRSGTDFTLTISSLQPEDFATYYCQQHYT TPPTFGQGTKVEIKRTVAAPSVFIFPPSDEQ LKSGTASVVCLLNNFYPREAKVQWKVDNALQ SGNSQESVTEQDSKDSTYSLSSTLTLSKADY EKHKVYACEVTHQGLSSPVTKSFNRGEC |
| 3 | Second heavy chain variable region (Trastuzumab) WT | |
| 4 | First heavy chain variable region (Pertuzumab) WT | |
| 5 | First heavy chain HCDR1 (Pertuzumab) WT | AASGFTFTDYTMD |
| 6 | First heavy chain HCDR2 (Pertuzumab) WT | DVNPNSGGSIYNQRFKG |
| 7 | First heavy chain HCDR2 (Pertuzumab) variant 1 | DVNPNSGGSILNRRFKG |
| 8 | First heavy chain HCDR2 (Pertuzumab) variant 2 | DVNPNSGGSILNVRFKG |
| 9 | First heavy chain HCDR2 (Pertuzumab) variant 3 | DVNPNSGGSIFNQRFKG |
| 10 | First heavy chain HCDR2 (Pertuzumab) variant 4 | DVNPNSGGSIFNHRFKG |
| 11 | First heavy chain HCDR2 (Pertuzumab) variant 5 | DVNPNSGGSIINQRFKG |
| 12 | First heavy chain HCDR2 (Pertuzumab) variant 6 | DVNPNSGGSIMNQRFKG |
| 13 | First heavy chain HCDR2 (Pertuzumab) variant 7 | DVNPNSGGSITNQRFKG |
| 14 | First heavy chain HCDR3 (Pertuzumab) WT | ARNLGPSFYFDY |
| 15 | First heavy chain HCDR3 (Pertuzumab) variant 1 | ARNLGPWFYFDY |
| 16 | First heavy chain HCDR3 (Pertuzumab) variant 2 | ARNLGPNFYFDY |
| 17 | First heavy chain HCDR3 (Pertuzumab) variant 3 | ARNLGPFFYFDY |
| 18 | First heavy chain HCDR3 (Pertuzumab) variant 4 | ARNLGPLFYFDY |
| 19 | First heavy chain HCDR3 (Pertuzumab) variant 5 | ARNLGPYFYFDY |

Although the specific embodiments of the present invention have been described above, it is to be understood by those skilled in the art that these are only provided by way of example, and various changes or modifications can be made to these embodiments without departing from the principle and essence of the present invention. Accordingly, the scope of protection of the present invention is defined by the appended claims.

## Claims

1. An anti-HER2 bispecific antibody, comprising a first protein functional region and a second protein functional region for respectively recognizing extracellular domain II and extracellular domain IV of HER2, wherein the first protein functional region comprises a first heavy chain and a first light chain, and the second protein functional region comprises a second heavy chain and a second light chain, wherein the first heavy chain comprises a heavy chain variable region as shown in the amino acid sequence of SEQ ID NO: 4 or a mutant thereof, the second heavy chain comprises a heavy chain variable region as shown in the amino acid sequence of SEQ ID NO: 3 or a mutant thereof, and the light chains comprise a light chain variable region comprising one or more amino acid substitutions at a position(s) selected from the following in the amino acid sequence set forth in SEQ ID NO: 1: R24, N30, T31, A32, F53, L54, S56, R66, H91, T93, T94 and P96.

2. The bispecific antibody of claim 1, wherein in the light chain variable region of the bispecific antibody, the amino acid substitution(s) is/are to substitute the original amino acid residue with an amino acid residue selected from the group consisting of: D, E, S, T, N, Q, G, A, V, I, L, K, M, F, Y, W and R.

3. The bispecific antibody of claim 1, wherein the light chain variable region of the bispecific antibody comprises one or more of the following amino acid residue substitutions in the amino acid sequence set forth in SEQ ID NO:1: R24K, N30S, T31I, A32G, F53Y, L54R, S56T, R66G, H91Y, T93I, T94Y and P96Y; and preferably one or more of the following amino acid residue substitutions: N30S, F53Y, L54R, S56T and P96Y.

4. The bispecific antibody of claim 1, wherein the light chain variable region of the bispecific antibody comprises one or more amino acid residue substitutions in the amino acid sequence set forth in SEQ ID NO: 1 selected from the group consisting of: (1) F53Y; (2) F53Y, L54R and S56T; (3) P96Y; (4) N30S and F53Y; (5) N30S, F53Y, L54R and S56T; or (6) N30S and P96Y.

5. The bispecific antibody of claim 1, wherein the bispecific antibody further comprises a light chain constant region, preferably a human lambda or kappa light chain constant region; and preferably, the light chain comprises one or more amino acid residue substitutions in the amino acid sequence set forth in SEQ ID NO: 2 selected from the group consisting of: (1) F53Y; (2) F53Y, L54R and S56T; (3) P96Y; (4) N30S and F53Y; (5) N30S, F53Y, L54R and S56T; or (6) N30S and P96Y.

6. The bispecific antibody of claim 1, wherein the first heavy chain and the second heavy chain of the bispecific antibody further comprise a heavy chain constant region, preferably a human heavy chain constant region; preferably, the first heavy chain and the second heavy chain of the bispecific antibody form a heterodimer preferably through linking via a "Knob-in-Hole" structure; and more preferably, the heavy chain variable region of the second heavy chain comprises mutation D102E in the amino acid sequence as set forth in SEQ ID NO: 3.

7. The bispecific antibody of any one of claims 1-6, wherein the heavy chain variable region of the first heavy chain of the bispecific antibody comprises the following CDR sequences: HCDR1 as shown in SEQ ID NO: 5, HCDR2 as shown in SEQ ID NOs: 6-13, and HCDR3 as shown in SEQ ID NOs: 14-19; and
preferably, the heavy chain variable region of the first heavy chain comprises HCDR1 as shown in SEQ ID NO: 5, HCDR2 as shown in SEQ ID NO: 6, and HCDR3 as shown in SEQ ID NO: 15; or HCDR1 as shown in SEQ ID NO: 5, HCDR2 as shown in SEQ ID NO: 7, and HCDR3 as shown in SEQ ID NO: 16; or HCDR1 as shown in SEQ ID NO: 5, HCDR2 as shown in SEQ ID NO: 8, and HCDR3 as shown in SEQ ID NO: 15; or HCDR1 as shown in SEQ ID NO: 5, HCDR2 as shown in SEQ ID NO: 9, and HCDR3 as shown in SEQ ID NO: 15; or HCDR1 as shown in SEQ ID NO: 5, HCDR2 as shown in SEQ ID NO: 10, and HCDR3 as shown in SEQ ID NO: 15; or HCDR1 as shown in SEQ ID NO: 5, HCDR2 as shown in SEQ ID NO: 7, and HCDR3 as shown in SEQ ID NO: 17; or HCDR1 as shown in SEQ ID NO: 5, HCDR2 as shown in SEQ ID NO: 11, and HCDR3 as shown in SEQ ID NO: 17; or HCDR1 as shown in SEQ ID NO: 5, HCDR2 as shown in SEQ ID NO: 12, and HCDR3 as shown in SEQ ID NO: 17; or HCDR1 as shown in SEQ ID NO: 5, HCDR2 as shown in SEQ ID NO: 13, and HCDR3 as shown in SEQ ID NO: 17; or HCDR1 as shown in SEQ ID NO: 5, HCDR2 as shown in SEQ ID NO: 7, and HCDR3 as shown in SEQ ID NO: 18; or HCDR1 as shown in SEQ ID NO: 5, HCDR2 as shown in SEQ ID NO: 7, and HCDR3 as shown in SEQ ID NO: 19.

8. The bispecific antibody of any one of claims 1-7, wherein the bispecific antibody has a reduced fucose modification; and preferably, the antibody is free of fucose modification.

9. A nucleic acid sequence encoding the bispecific antibody or an antigen-binding portion thereof of any one of claims 1-7.

10. An expression vector, comprising the nucleic acid sequence of claim 9.

11. A prokaryotic or eukaryotic cell, comprising the expression vector of claim 10.

12. A light chain, comprising a light chain variable region, wherein the light chain variable region comprises one or more amino acid residue substitutions in the amino acid sequence set forth in SEQ ID NO: 1 selected from the group consisting of: (1) F53Y; (2) F53Y, L54R and S56T; (3) P96Y; (4) N30S and F53Y; (5) N30S, F53Y, L54R and S56T; or (6) N30S and P96Y; preferably, the light chain further comprises a light chain constant region, preferably a human lambda or kappa light chain constant region; and more preferably, the light chain comprises one or more amino acid residue substitutions in the amino acid sequence set forth in SEQ ID NO: 2 selected from the group consisting of: (1) F53Y; (2) F53Y, L54R and S56T; (3) P96Y; (4) N30S and F53Y; (5) N30S, F53Y, L54R and S56T; or (6) N30S and P96Y.

13. An application of the light chain of claim 12 in the preparation of an antibody, wherein preferably, the antibody is scFv, Fab', F(ab)2 or a full-length antibody, or a monoclonal antibody, a bispecific antibody or a multispecific antibody.

14. An anti-HER2 bispecific antibody-drug conjugate, prepared by covalently coupling the bispecific antibody of any one of claims 1-8 to a cytotoxic drug;
preferably, the cytotoxic drug is a tubulin inhibitor, a topoisomerase inhibitor or a DNA minor groove inhibitor; and
more preferably, the tubulin inhibitor is selected from maytansines, auristatin derivatives, or tubulin; the topoisomerase inhibitor is selected from DXd or derivatives thereof, SN-38, or doxorubicin and derivatives thereof; and the DNA minor groove inhibitor is selected from calicheamicin, duocarmycin and derivatives thereof, pyrrolobenzodiazepines or indolinobenzodiazepines.

15. A pharmaceutical composition comprising the bispecific antibody of any one of claims 1-8, wherein preferably, the pharmaceutical composition further comprises a hyaluronidase; more preferably, the hyaluronidase is rHuPH20 or modified rHuPH20; and most preferably, the pharmaceutical composition further comprises a pharmaceutically acceptable carrier or excipient.

16. A method for preparing the bispecific antibody of any one of claims 1-8, comprising the steps of:
(1) constructing the nucleotide sequences encoding the heavy chains and the nucleotide sequence encoding the light chains in the same vector or different vectors; and
(2) expressing the vector(s) constructed in the step above in different host cells or the same host cell.

17. An application of the anti-HER2 bispecific antibody of any one of claims 1-8, the light chain of claim 12 or 13, the anti-HER2 bispecific antibody-drug conjugate of claim 14 and the pharmaceutical composition comprising the bispecific antibody of claim 15 in the preparation of a medicament for treating HER2-expressing tumors, wherein preferably, the positive tumors comprise breast cancer, gastric cancer, prostate cancer, lung cancer, bladder cancer, ovarian cancer, colon cancer, esophageal cancer, and head and neck cancer, endometrial cancer, malignant melanoma, pharyngeal cancer, oral cancer and skin cancer; and more preferably, breast cancer, gastric cancer and lung cancer.
